# EUROPEAN PATENT APPLICATION

(11) **EP 4 495 945 A1**
(43) Date of publication of application: **22.01.2025**
(21) Application number: 23186672.4
(22) Date of filing: 20.07.2023
(51) Int. Cl.: G16H 40/63, A61B 6/00, G16H 50/20, G01R 33/54

(54) **MANIPULATING A MEDICAL IMAGE ACQUISITION SYSTEM**

(71) Applicant: Koninklijke Philips N.V., 5656 AG Eindhoven (NL)
(72) Inventor: HAHN, Artur, Eindhoven (NL)
(74) Representative: Philips Intellectual Property & Standards

(57) **Abstract**

A computer-implemented method (100) to manipulate a medical image acquisition system comprises using a large language model to receive (101) inputs and determine (102) instructions based on the received inputs. The inputs comprise a user defined input comprising any one or a combination of a medical imaging exam type; a plurality of symptoms; and an instruction to manipulate the medical image acquisition system. The determined outputs comprise instructions comprising a first position instruction for the image acquisition sensor with respect to a subject; anda first plurality of medical image acquisition system settings, wherein the first plurality of medical image acquisition system settings comprises at least image acquisition sensor settings.

## Description

### FIELD OF THE INVENTION

The invention relates to manipulating a medical image acquisition system.

### BACKGROUND OF THE INVENTION

Medical imaging exams are typically performed by imaging specialists requiring years of education and experience. To alleviate the education and training needs, imaging exam guidance methods have been presented. However, none of these methods allow entirely unskilled people to operate the medical image acquisition devices.

Current solutions to improve the workflow, for example adaptively recommend and/or select a next task following completion of a current task as disclosed in for example US 8355928 B2.

### SUMMARY OF THE INVENTION

It is, inter alia, an object of the invention to simply the operation of a medical imaging system. The invention provides instructions to an operator of a medical imaging device to operate said medical imaging device. In particular, it is envisioned by the inventors to provide instructions using a large language model, wherein the instructions are so clear to the operator, that the subject being imaged could operate the medical image acquisition device. Thereby enabling self-operation of medical image acquisition systems during medical imaging exams. In a preferred embodiment, the invention is envisioned to be implemented in ultrasound systems, which are safe and relatively easy to use outside of hospital environments.

The invention is defined by the independent claims. Advantageous embodiments are defined in the dependent claims.

According to an aspect of the invention there is provided a method to manipulate a medical image acquisition system comprising an image acquisition sensor, the method comprising using a large language model configured to:
- receive a user input, wherein the user input comprises any one of:
   - a medical imaging exam type;
   - a plurality of symptoms; and
   - an instruction to manipulate the medical image acquisition system; and
- based on the user input, determine
   - a first position instruction for the image acquisition sensor with respect to a subject; and
   - a first plurality of medical image acquisition system settings, wherein the first plurality of medical image acquisition system settings comprises at least image acquisition sensor settings.

In this manner, the method enables a subject to receive instructions to place the imaging sensor in a desired location according to the first position, and to configure the medical image acquisition system to the desired system settings according to the first plurality of medical image acquisition system settings. As a result of this help, a subject may be able to self-operate a medical image acquisition system freeing up time of physicians (e.g., sonographers, radiologists, etc.).

In an example the method further comprises
- conveying the first position instruction for the image acquisition sensor to a user; and/or
- moving the imaging sensor to the first position. For example, via an automatic arm or gantry.

In an example the large language model is further configured to:
- responsive to the image acquisition sensor being positioned at the first position:
   - determine an instruction to acquire a medical image;
wherein the method optionally further comprises to acquire the medical image according to the instruction to acquire the medical image.

In this manner, once the imaging sensor is placed at the desired location, either manually by an operator or the subject, or automatically, the medical image acquisition system may be controlled to automatically acquire a medical image. Thereby facilitating the workflow.

In an example, the large language model is further configured to:
- responsive to receiving the acquired medical image:
   - determine a second plurality of medical image acquisition system settings,
      wherein the second plurality of medical image acquisition system settings comprises at least image acquisition sensor; and/or
   - determine a second position instruction for the image acquisition sensor with respect to the subject.

In this manner, the imaging parameters of the system and/or sensor may be updated for a potential second image. For example, by determining parameters for a different view of the imaged feature or to improve the image quality by adjusting the imaging settings.

In an example, wherein the large language model is further configured to:
- generate a medical examination report

In an example, the examination report is based on at least any one or a combination of:
- the user input;
- the medical image;
- a medical report template; and
- a medical proficiency level of a potential recipient.

In this manner, the imaged subject can receive an immediate summary of the medical findings, while a full report of the findings may also be available to be sent to the physician treating the subject.

In an example, the large language model is further configured to:
- responsive to the image acquisition sensor being positioned at the first position:
   - receive a pressure signal representing a pressure between the image acquisition sensor and the surface of the subject, and based on the received pressure signal, determine a pressure instruction; and/or
   - receive a moisture signal representing moisture at the image acquisition sensor, and based on the received moisture signal, determine a gel fluid application instruction.

In this manner, it may for example be determined whether an ultrasound probe is in contact with a surface of a subject and/or whether a right amount of pressure is applied with the ultrasound probe on the surface of the subject. Based on the received pressure signal, an instruction to increase, reduce or keep the pressure may be given. The instruction may even detail to apply more pressure to a particular part of the probe, while reducing it at others. Further, in this manner it may be determined whether sufficient acoustic gel-fluid is used at the ultrasound probe and subject surface interface. Based on the received moisture signal, an instruction may be given to for example apply more gel-fluid, to remove some gel-fluid, or to switch to a gel-pad.

In an example, the large language model is further configured to:
- receive positioning data of the medical image acquisition sensor with respect to the subject; and any one or a combination of the instructions are further based on the positioning data of the medical image acquisition sensor with respect to the subject. For example:
   - the first position instruction for the image acquisition sensor with respect to a subject;
   - the first plurality of medical image acquisition system settings;
   - the second position instruction for the image acquisition sensor with respect to a subject; and/or
   - the second plurality of medical image acquisition system settings;
   may be further based on the positioning data of the medical image acquisition sensor with respect to the subject.

In this manner, the position of the imaging sensor and subject may be determined more accurately to further improve the positioning guidance of the sensor and/or the subject. The positioning guidance, according to this example is thus not based purely on an ideal or learned position, but may be based on the current real-time position of the imaging sensor with respect to the subject.

In an example, the large language model is trained through unsupervised learning, wherein unsupervised learning comprises:
- receiving an unlabeled data set comprising a plurality of instances, each instance including one or more features; and
- initializing the large language model with adjustable parameters;
- applying an unsupervised learning algorithm to the data set using the large language model, wherein the unsupervised learning algorithm modifies the adjustable parameters of the large language model based on relationships between the features in the instances without referring to any predetermined label or outcome and wherein the modification of the adjustable parameters is performed iteratively until a stopping criterion is met. Thereby training the large language model general knowledge, including for example a full medicine study and vast amounts of medical reports.

In an example, the large language model is trained through supervised learning, wherein supervised learning comprises:
- receiving a labeled dataset comprising a plurality of instances, each instance comprising an input feature and an associated output feature;
- initializing the large language model with adjustable parameters; and
- applying a supervised learning algorithm to the labeled dataset using the large language model, wherein the supervised learning algorithm iteratively modifies the adjustable parameters of the large language model based on a comparison between the large language model prediction given the input feature and the associated output feature until a predetermined stopping criterion is satisfied. Thereby training the large language model particular medical routines and practices regarding medical imaging exams.

In an example, the labeled data set comprises medical imaging acquisition exams, wherein, in each instance, the input feature comprises the received data or information of method 100, together also named as inputs throughout this specification. In particular, the inputs may be:
- the user input; and
- optionally:
   - the pressure,
   - the moisture and
   - the medical image; and
the associated output comprises the determined instructions of method 100, together also named as outputs throughout this specification. In particular:
- the first position instruction for the image acquisition sensor with respect to a subject;
- the first plurality of medical image acquisition system settings; and
- optionally:
   - the instruction to acquire a medical image;
   - the second plurality of medical image acquisition system settings;
   - the second position instruction for the image acquisition sensor with respect to the subject;
   - the medical examination report;
   - the pressure instruction;
   - the gel-fluid application instruction.

In an example, the large language model is trained through reinforcement learning, wherein reinforcement learning comprises:
- initializing the large language model with adjustable parameters;
- applying a reinforcement learning algorithm, wherein the large language model interacts with an environment, performs actions based on its current state and parameters, and receives rewards or penalties based on the performed actions; and
- iteratively adjusting the model parameters based on the received rewards or penalties until a predetermined stopping criterion is met. In this manner, the large language model can be trained to comply with given safety bounds and to not provide instructions outside of these bounds.

In an example, the interaction of the large language model with the environment comprises instructing, configuring, and performing medical imaging examinations according to the method as disclosed herein, and wherein the rewards or penalties based on the performed actions are based on a user defined performance or based on a pre-defined loss function.

In accordance with another aspect of the invention there is provided a computer program product comprising instructions, which when executed by a processor, cause the processor to perform any of the methods disclosed herein.

In accordance with yet another aspect of the invention there is provided a medical image acquisition system comprising:
- a medical image acquisition sensor;
- a user interface; and
- a processor in communication with the medical image acquisition sensor and the user interface, the processor being configured to perform any of the methods disclosed herein.

In an example, the medical image acquisition system comprises an ultrasound system and the system further comprises:
- a pressure sensor configured to determine the pressure between the ultrasound transducer array and a surface of the subject; and
- a moisture sensor configured to determine the moisture between the ultrasound transducer array and the surface of the subject.

According to an embodiment of the invention there is provided a (smart and self-operable) ultrasound system comprising:
- an ultrasound probe comprising;
   - a transducer array configured to emit and receive acoustic signals;
- a user interface configured to:
   - receive (100) a user input, wherein the user input comprises any one of:
      - a medical imaging exam type;
      - a plurality of symptoms; and
      - an instruction to manipulate the medical image acquisition system;
   and
- a processor in communication with the ultrasound probe and the user interface, the processor configured to run a large language model that based on the user input, determines
   - a first position instruction for the image acquisition sensor with respect to a subject; and
   - a first plurality of medical image acquisition system settings, wherein the first plurality of medical image acquisition system settings comprises at least image acquisition sensor settings.

In an example, the ultrasound probe further comprises a pressure sensor to determine the position of the ultrasound probe with respect to the subject. For example, whether the ultrasound probe is located on the subject's skin. For example, whether the right amount of pressure is applied on the subject's skin. The pressure sensor may be implemented as an algorithm by determining tissue and/or gel-pad deformation. In such an example, the pressure exerted by the probe on the subject may be received without a pressure sensor.

In an example, the ultrasound system may further comprise a temperature sensor to determine whether the ultrasound probe is in contact with a subject's skin. For example, based on the temperature sensed at the subject's skin.

In an example, the ultrasound probe further comprises a moisture sensor to determine whether sufficient gel-fluid is present between the ultrasound transducer array and the subject's skin.

In an example, the ultrasound system is further configured to generate a medical image based on the emitted and received acoustic signals. For example, the ultrasound system may further comprise a beamformer and/or a processor configured to process the acoustic signals and generate an ultrasound image.

In an example, the ultrasound processor is further configured to control the ultrasound probe based on the determined outputs.

In an example, the user interface is further configured to convey the determined outputs to the user and/or subject of the ultrasound system.

These and other aspects of the invention will be apparent from and elucidated with reference to the embodiments described hereinafter.

### BRIEF DESCRIPTION OF THE DRAWINGS

Fig. 1 shows a flow chart according to an embodiment of the invention.
Fig. 2 shows a schematic of a medical image acquisition system according to an embodiment of the invention.
Fig. 3 shows a schematic of an ultrasound imaging system according to an embodiment of the invention.
Fig. 4 shows a schematic of a processor according to an embodiment of the invention.

### DESCRIPTION OF EMBODIMENTS

The invention will be described herein with reference to the Figures. It should be understood that the description and specific examples provided, while indicating exemplary embodiments, are intended for purposes of illustration only, and not intended to limit the scope of the invention. It should also be understood that the figures are mere schematics and not drawn to scale. It should also be understood that the same reference numerals are used throughout the Figures to indicate the same or similar parts.

The invention provides a computer-implemented method to operate a medical image acquisition system.

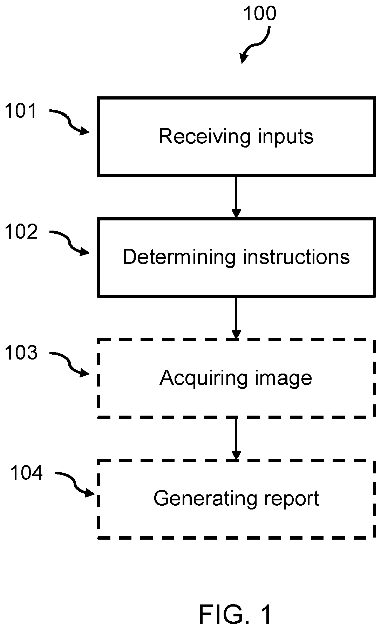

Fig. 1 shows an exemplary flow chart according to a method of the invention. The method 100 as described in Fig. 1 comprises a large language model configured to receive 101 a (free-text) user input, wherein the user input comprises any one of:
- a medical imaging exam type, for example X-ray imaging, ultrasound, Magnetic Resonance (MR) imaging, Computed Tomography (CT) imaging, or specifically lung X-ray exam, 20-week fetal ultrasound exam, brain magnetic resonance exam, mammography, dental imaging, etc.;
- a plurality of symptoms, for example persistent headache, sudden or unexpected discomfort in any part of the body, bone pain or fractures, cognitive or behavioral changes prolonged cough, discomfort around the heart, discomfort around the liver region, etc.; and
- an instruction to manipulate a medical image acquisition system. For example, the subject may be a more experienced subject and may indicate system settings for the desired medical imaging system. For example, the subject may indicate a desired radiation strength for an X-ray imaging exam, a desired type of probe for an ultrasound exam, desired imaging sensor configuration settings, such as depth or aperture in ultrasound, etc.

The large language model may further receive a first plurality of configuration settings of the medical image acquisition system, the medical image acquisition system comprising a medical imaging sensor. In other words, the method according to the invention may receive a current state of the medical image acquisition system, wherein the current state may comprise any one of: image configuration settings, for example, radiation strength in X-ray, image depth, aperture, etc. in ultrasound, etc.; and

The large language model may further receive positioning data of the medical imaging sensor with respect to a subject. The positioning data may be as simple as medical imaging acquisition sensor on the subject or not, medical imaging acquisition sensor imaging the heart or medical imaging acquisition sensor imaging empty space, etc. Additionally or alternatively, the method may receive information with regard to the position and/or posture of the subject with respect to the imaging sensor of the medical image acquisition system. The positioning data of the subject and/or medical imaging sensor may be received from for example cameras (e.g., optical cameras, infrared cameras, depth cameras (RGB(D) cameras) or any other suitable camera) designed to track and monitor the subject and/or the imaging sensor right before, during and/or right after a medical imaging exam. These cameras may further be in communication with or be a part of a medical image acquisition system. For example, method 100 may receive information with regard to the location of an X-ray source and detector of an X-ray imaging system. For example, method 100 may receive information with regard to the position of an ultrasound probe of an ultrasound system, including but not limited to whether the ultrasound probe is still in the holder, is located on the surface of the subject, is internal to the subject, etc. Additionally or alternatively to cameras, it is possible to determine for example an ultrasound probe location based on a medical image acquired by said probe. For example, if the probe is imaging a heart from a particular angle it may be located on the chest of the subject. For example, based on a detected pressure from a pressure sensor it is possible to determine that the medical imaging sensor (e.g., ultrasound probe) is in contact with a surface of the subject (e.g., the subject's skin). For example, based on a temperature it is possible to determine whether the imaging sensor is in contact with a surface of a subject (e.g., the subject's skin).

In a second step, method 100, based on the user input, determines 102 instructions comprising:
- a first position instruction for the image acquisition sensor with respect to a subject; and
- a first plurality of medical image acquisition system settings, wherein the first plurality of medical image acquisition system settings comprises at least image acquisition sensor settings.

In an example these instructions may be used to
- conveying the first position instruction for the image acquisition sensor with respect to the subject to a user; and/or
- moving the imaging sensor to the first position.

For example, guidance may be provided to an ultrasound system operator with regard to the placement of the ultrasound probe. In an example, the operator may also be the subject. For example, method 100 may control for example a gantry of an X-ray system to position the X-ray source and/or detector. For example method 100 may be in communication with a robotic arm (e.g., as disclosed in WO 2019/175129) capable of handling an ultrasound probe.

The instructions determined according to method 100 may further comprise a position guidance for the subject. For example, image, text or spoken instructions to the subject, for the subject to be positioned to have a medical image taken. In case of an MR imaging scan, this may involve instructing the subject to remove all metal objects, to remove certain type of clothes, to lie on the MR bed, breathing instructions, pushing particular buttons, etc. In case of an ultrasound imaging exam, this may involve instructions on holding the ultrasound probe, placing the ultrasound probe, breathing instructions, pressure applied by the ultrasound probe on the subject's surface, etc.

In an example, the large language model is further configured to:
- responsive to the image acquisition sensor being positioned at the first position:
   - determine (103) an instruction to acquire a medical image;
wherein the method optionally further comprises acquiring the medical image according to the instruction to acquire the medical image.

In other words, manual, semi-automatic or automatic operation may be enabled to operate and control 103 a medical image acquisition system.

For example, in automatic operation, the X-ray source and/or detector could be moved automatically along gantry according to a position instruction and the radiation dose may be selected based on the user input and the determined plurality of medical image acquisition system settings.

For example, in manual operation, an ultrasound system operator may receive instructions on where to place the probe, what image settings to use, etc. These instructions may be given in any suitable form, including but not limited to text instructions, audible instructions, tactile instructions or any other form of instructions. According to method 100, at least part of the instructions may thus be conveyed to a user (or the subject) to operate the imaging acquisition sensor.

For example, in semi-manual operation, an ultrasound system operator may be instructed where to place the ultrasound probe, while method 100 controls the ultrasound imaging system to acquire a medical image according to the determined plurality of medical image acquisition system settings. In examples, the operator of the medical image acquisition system may also be the subject whose image will be taken. Thereby enabling self-imaging in medical imaging. In other words, the subject may also be the operator.

In an example, the large language model is further configured to:
- responsive to receiving the acquired medical image:
   - determine a second plurality of medical image acquisition system settings,
      wherein the second plurality of medical image acquisition system settings comprises at least image acquisition sensor; and/or
   - determine a second position instruction for the image acquisition sensor with respect to the subject.

In this manner, method 100 may thus automatically adjust the instructions given to the operator (whether subject or practitioner) based on the received medical image or medical imaging data. Thereby enabling guidance during a medical imaging exam. This is seen as particularly advantageous in ultrasound exams, where a continuous image feed is obtained through continuous physical interaction between the ultrasound imaging sensor tip (i.e., the ultrasound probe) and the subject. Nonetheless, also in for example MR exams this may be advantageous by providing instructions on modifying the gradient fields (i.e., the rapidly changing magnetic fields superimposed on the main static magnetic field) and/or radiofrequency pulses during the imaging procedure.

In an example, the large language model is further configured to:
- generate 104 a medical examination report, wherein the examination report. This medical examination report may be generated according to for example US 63/463,709 (Earl Canfield). US 63/463,709 discloses to for example to generate a diagnostic report given an input data string. This input data may for example be fetal measurements, and the diagnostic report may comprise a summary of the fetal measurements, medical images to the measurements, findings derived from the measurements, etc. while at the same time being able to format the report according to a desired standard. Said standard being defined for example by a particular hospital and input to the method 100 as the standard to be followed. In other examples, the medical examination report may simply comprise a list of findings in a direct communication (e.g., an audio summary or a print-out) to the operator and/or subject immediately after the medical examination. This list of findings may be tailored to the user inputs (e.g., the list of symptoms) or include all findings, also those not related to the user inputs.

The examination report may be based on processing the medical images to identify and determine any findings. This processing may be done by a large language model algorithm.

In an example, the medical examination report is based on at least any one or a combination of:
- the user input;
- the medical image;
- a medical report template; and
- a medical proficiency level of a potential recipient.

The medical report template may for example be a user defined or specific template, a pre-defined based on the medical examination type, or a template to comply with particular hospital and/or insurance requirements for the medical report.

A medical examination report for an expert, a physician, doctor, sonographer, etc., may say:
'The study provided good quality images with adequate visualization of the cardiovascular structures.

The left ventricle (LV) demonstrated mild hypertrophy and systolic function was mildly reduced, with an ejection fraction estimated at 45%. The left atrium was mildly dilated.

The right ventricle (RV) was normal in size and function. The right atrium was unremarkable.

Valvular structures showed mild mitral regurgitation. The aortic, tricuspid, and pulmonic valves displayed no significant stenosis or regurgitation. The interatrial and interventricular septa were intact.

Evaluation of the coronary arteries demonstrated the presence of significant echogenic material consistent with atheromatous plaques in the proximal and mid segments of the left anterior descending (LAD) artery. The plaques cause approximately 70% luminal narrowing. The circumflex (LCX) and right coronary artery (RCA) showed mild atherosclerotic changes.

Doppler studies revealed impaired coronary flow reserve in the LAD territory, suggesting hemodynamically significant stenosis. 2D strain imaging showed mild regional wall motion abnormalities in the anterior wall, indicating likely subendocardial ischemia.'

In contrast, a similar report for the subject or a person of lower medical proficiency may say:
'The pictures we took of the heart were clear and showed us what we needed to see.

The main pumping chamber of the heart (the left ventricle) is a bit thicker than normal, and it's not pumping blood as well as it should be - it's working at about 45% of its capacity. The top left chamber (the left atrium) is slightly bigger than normal.

The right side of the heart looks fine.

One of the valves in the heart is leaking a bit, allowing some blood to flow backward.

The blood vessels that supply the heart muscle (coronary arteries), show some build-up, similar to rust in a pipe, in the artery called the left anterior descending artery (LAD). This build-up is blocking about 70% of the artery. There's also a little bit of this build-up in the other two main arteries supplying the heart.

The blood flow in the LAD is impaired, suggesting that the blockage is significant. Also, a portion of the heart muscle isn't moving as well as it should be, which can happen when it's not getting enough blood and oxygen.'

In an example, the large language model is further configured to:
- responsive to the image acquisition sensor being positioned at the first position:
   - receive a pressure signal representing a pressure between the image acquisition sensor and the surface of the subject and based on the received pressure determine a pressure instruction; and/or
   - receive a moisture signal representing moisture at the image acquisition sensor and based on the received moisture determining a gel fluid application instruction.

For example, based on the pressure signal, it is possible to determine whether the imaging sensor is in contact with a subject's skin and/or to determine whether the right amount of pressure is applied to the subject's skin by the medical imaging sensor. This example is particularly envisioned for ultrasound transducer arrays as part of ultrasound systems. In an example the pressure is determined by a pressure sensor. In an example the pressure is determined based on tissue deformation and elasticity. In an example the pressure is determined based on a gel-pad deformation.

For example, based on the moisture signal, it may be determined whether enough gel-fluid is applied at the imaging sensor and subject's skin interface. This example is particularly envisioned in view of ultrasound systems.

In an example, method 100 further comprises receiving a temperature at the medical imaging sensor. For example, from a temperature sensor. In this manner, contact with a surface may be determined.

Method 100 is envisaged to be performed by a trained large language model. In a preferred example, a large language model comprising a transformer architecture is envisioned. Such a large language model may be substantially similar to the large language model behind ChatGPT. In an example, a pre-trained model such as GPT-4 may also be trained further as described herein to perform method 100.

A large language model architecture is a sophisticated and highly-scalable framework, designed to handle and generate human-like text based on input data. This model is developed around a transformer-based architecture, a machine learning structure, primarily designed to process sequential data.

The transformer-based architecture consists of an encoder that reads the input data and a decoder that produces the output. However, for large language models, emphasis is often placed on the decoder component. This decoder, constructed with a stack of identical layers, is known to be very suitable for language modelling tasks where the objective is to predict the next element in a sequence, such as the next word in a sentence.

One of the pivotal features of the architecture is the self-attention mechanism. This mechanism allows the model to weight the importance of each element within an input sequence when predicting the subsequent element. With multi-head attention, several such relational weighting sets are used. Consequently, it can comprehend the context and dependencies between individual elements, thereby providing improved prediction accuracy.

The model is organized into multiple layers. Each layer includes a multi-headed self-attention mechanism and a position-wise feed-forward network. These layers can house an extensive array of (adjustable) parameters, potentially numbering into the billions. The high number of parameters facilitates the model's ability to capture and interpret intricate patterns and subtle nuances within the processed data.

Additionally, the model integrates a feature referred to as positional encoding. Which enables injecting information about the relative positions or the sequence of elements within for example a sentence. In other words, providing context.

Training of the large language model may comprise any one or a combination of:
- unsupervised learning;
- supervised learning; and
- reinforcement learning.

In an example, training the large language model according to unsupervised learning comprises:
- receiving an unlabeled data set comprising a plurality of instances, each instance including one or more features;
- initializing the large language model with adjustable parameters; and
- applying an unsupervised learning algorithm to the data set using the large language model, wherein the unsupervised learning algorithm modifies the adjustable parameters of the large language model based on relationships between the features in the instances without referring to any predetermined label or outcome and wherein the modification of the adjustable parameters is performed iteratively until a stopping criterion is met. The objective of the optimization algorithm tuning the parameters is to maximize the probability of predicting the next word in a sequence of inputs, which is iteratively done while incrementally crawling through text material, word for word.

Upon meeting the stopping criterion, the training according to unsupervised learning may be considered completed. The result at this point is then a trained large language model, wherein the trained large language model is configured to accept new instances of data and generate an output based on the adjusted parameters and the relationships learned during the unsupervised learning process, wherein the output provides an analysis or categorization of the new instance of data and generates outputs based on the most likely next word.

In the preferred embodiment wherein the large language model is a large language model, unsupervised learning typically comprises training on an unlabeled data set according to the above description with the aim of predicting the next most probable token in a series of tokens (e.g., a word in a series of words) given to the model. In other words, the trained large language model will predict in each a first word, given the first word a second word, given the first two words the third word, and so on, to generate full sentences, paragraphs and finally texts. By learning the inference of the next words of a sentence or conversation, a transformer architecture of a large language model (e.g., ChatGPT) attains general semantic knowledge.

For a large language model to be used to perform the steps of method 100, it is thus advantageous to train the large language model on unlabeled data sets to learn general knowledge and the ability to paraphrase information in different ways, but it may also help to include data sets specific about human anatomy, diseases, diagnostic possibilities, etc. These data sets may be attained for example from medical textbooks and/or medical reports used in for example training medicine students (e.g., at a university) or doctors (e.g., at a hospital).

Suitable unsupervised learning algorithms may be for example clustering, dimensionality reduction, anomaly detection. These algorithms are considered known to the skilled person.

Suitable data for unsupervised learning is readily available on the world wide web and is known by the skilled person in the art.

In an example, training the large language model or large language model according to supervised learning comprises:
- receiving a labeled dataset comprising a plurality of instances, each instance comprising an input feature and an associated output feature.
- initializing the large language model with adjustable parameters; and
- applying a supervised learning algorithm to the labeled dataset using the large language model, wherein the supervised learning algorithm iteratively modifies the adjustable parameters of the large language model based on a comparison between the large language model prediction given the input feature and the associated output feature until a predetermined stopping criterion is satisfied.

Upon meeting the stopping criterion, the training according to supervised learning may be considered completed. The result at this point is then a trained large language model, wherein the trained large language model is configured to process new data instances and predict a corresponding output based on the learned relationships between the input features and the associated output features that make up the plurality of instances. The relationships being displayed in the weights of the adjustable parameters.

The supervised learning algorithm may be for example any one or a combination of: regression, classification, support vector machines, decision trees, neural networks, or diffusion models. These algorithms are considered state of the art and known to the skilled person.

While a large language model may be trained immediately following the supervised learning routine, it is typical to use the large language model trained according to unsupervised learning as starting point to further train according to supervised learning. The step of supervised learning is thus commonly performed after the step of unsupervised learning.

In an example, the labeled data set comprises medical imaging acquisition exams, wherein, in each instance, the input feature comprises the received data, information or parameters of method 100 and the associated output features comprises the determined parameters of method 100.
For example, the input features comprises
- the user input; and
- optionally:
   - the pressure;
   - the moisture;
   - the medical image;
   - the first position instruction for the image acquisition sensor with respect to a subject; and
   - the first plurality of medical image acquisition system settings the associated output comprises:
- the first position instruction for the image acquisition sensor with respect to a subject;
- the first plurality of medical image acquisition system settings; and
- optionally:
   - the instruction to acquire a medical image;
   - the second plurality of medical image acquisition system settings;
   - the second position instruction for the image acquisition sensor with respect to the subject;
   - the medical examination report;
   - the pressure instruction;
   - the gel-fluid application instruction;
   - the second position instruction for the image acquisition sensor with respect to a subject; and
   - the second plurality of medical image acquisition system settings.

The labeled data set may for example be gathered by tracking and monitoring the interaction of an operator of a medical image acquisition system and a subject during a medical imaging exam.

The user input, e.g., the medical imaging exam type, the plurality of symptoms, the plurality of concerns and/or the instruction to manipulate the medical imaging acquisition system, may be given by a user via a user interface. For example, the user may speak into a microphone or type the inputs on a touch screen or keyboard.

The first and second plurality of configuration settings of the medical image acquisition system may be retrieved from a system log or database of preconfigured imaging settings options, or may be monitored in real time through software monitoring algorithms.

Data regarding positioning of the image acquisition sensor with respect to the subject may be obtained from an additional imaging sensor such as a camera. A suitable camera may for example be a RGB(D) camera imaging the entire or a part of a room in which the imaging procedure is to take place. Also examples in which a mobile phone or tablet camera can be used are envisaged herein. For example, method 100 may instruct the subject to take certain pictures or a video stream with their smart device which may serve as input for subject positioning data. Some X-ray systems already possess suitable cameras which are used for subject positioning guidance. All these camera systems may thus be suitable for tracking and monitoring the subject (in real-time). Additionally or alternatively, data regarding positioning of the imaging sensor may also be acquired from other sensors such as inertial measurement units, accelerometers, etc.

In an example, the position of the image acquisition sensor with respect to the subject may be received based on analyzing imaging data acquired with the imaging sensor. For example, when imaging a heart using an ultrasound probe, method 100 may receive the probe being located on the subject's skin imaging the heart. In an example where no relevant subject feature is imaged or where air is detected, the position of the probe with respect to the subject may be received as not being on the subject's skin. The position of the probe may also be received based on a pressure sensor sensing contact with the subject's skin.

A pressure or a temperature that a surface of a subject exerts on an ultrasound transducer array of an ultrasound probe may for example be obtained from a pressure sensor or a temperature sensor. The pressure may also be obtained based on tissue and/or gel-pad deformation. Based on the pressure and/or the temperature it can be determined whether the ultrasound probe is in contact with, and if at the right pressure, with the subject's skin.

A humidity or moisture at the interface of the imaging sensor and the subject's skin may be obtained from a humidity or moisture sensor.

The associated output data for training is gathered from a combination of software and sensor monitoring. For example, software may monitor an operator's actions on the medical image acquisition system, while sensors may monitor the actions and movements of the operator and/or the medical image acquisition sensor with respect to the subject. For example, microphones and cameras, as well as computer vision, segmentation, classification and filtering algorithms to extract the relevant information from the training material (like voice recordings of the trained physician, filtered by instructions during the imaging exam, explanations of what can be seen in the acquired image data, and diagnostic information or the interpretation of the image data, as well as recommendations on further actions or examinations).

In an example, keyboard trackers and other monitoring software may be used to monitor the input at the user interface. In this manner information regarding the medical imaging exam, system settings, annotations, measurements, etc. may be obtained. For example, the selected medical exam and/or a selected pre-defined work-flow may be determined through software monitoring.

In an example, imaging sensors such as RGB(D) cameras may be used to monitor the movement of the operator and/or the imaging sensor. In this manner, the large language model can for example learn the relationship between the imaging sensor position and the subject position and posture. In this manner, also the amount of applied gel-fluid in case of ultrasound examinations may be determined.

The associated output data may further be expanded. For example, an operator may be asked to speak out instructions (recorded via e.g., a microphone). For example, visual instructions may be prepared, for example images of imaging sensor with respect to subject placement. In examples, also other types of instructing material may be produced, like animations, projections, and/or augmented reality holograms, to be used as an associated output feature to given input features and to be able to provide better instructions according to step 102.

A simple manner to gather the associated output data without the need to expand the data with extra instructions, would be to allow a subject to self-operate the medical image acquisition system, while an operator is providing instructions to enable said self-operation. In this manner, the associated output data collected for training would already include the specific instructions to enable self-operation. In other words, an operator may not perform the medical image acquisition exam, but guide the subject to perform the exam while recording the guidance as the associated output data. The paraphrasing abilities of the trained large language model are beneficial here, in order to extract the semantic information of the instructions and be able to rephrase it according to the medical proficiency level of the user.

The associated output may further comprise the analysis of the medical images. In other words, relevant measurements, identification of broken bones, identification of tumors, etc. These diagnostic steps may be performed by the medical image acquisition system operator or by a physician after the imaging exam has taken place. Further, this data may be documented in a medical examination or findings report. Method 100 may also be taught to generate such a medical examination report by monitoring the operator and/or physician during the medical image analysis and report writing. Method 100 may thus automatically identify any medical findings, adjust the medical examination procedure, and generate a medical imaging report. The medical examination report may be adjusted based on specific user, hospital and/or insurance templates and requirements. The medical examination report may further be adjusted based on a perceived and/or selected medical proficiency of the desired recipient (e.g., the subject). In other words, the medical imaging report may be written in a style comprehensible by non-physicians. Expressing the medical examination report in various styles can be achieved via supervised learning, by providing these styles, but it may also be achieved via unsupervised learning, wherein the method 100 is for example asked to explain it to a subject as if they were 10 years old.

The associated output data may further be augmented by specifically prepared instruction material such as visual guidance models.

The medical image acquisition system settings may be retrieved from the system logs.

A large language model trained according to supervised learning on data as described above would thus naturally adhere to the medical imaging examination routines it has been trained on, and is thus fully enabled to perform method 100. However, given unexpected and/or deviating input to the input on which the large language model was trained on, the large language model may perform unexpectedly and unwantedly. A chance thus prevails of unreasonable and/or unrealistic outputs responsive to certain inputs. In order to overcome this the large language model may further be trained according to reinforcement learning.

Reinforcement learning is a type of learning in which a trained large language model is graded in use. For example, if the large language model performs as expected, it may be rewarded. If on the other hand the large language model performs against expectation and/or outside of its supposed bounds, it is penalized. This reward and punishment typically are performed via a pre-defined loss function. However, it may also be performed manually, where testers (e.g., operator used for testing method 100 or very experienced subjects) using medical image acquisition systems according to method 100 may provide a score after each use. In this manner, the large language model algorithm will adjust the weights to better conform with the testers wishes and/or expectations.

In other words, feedback on instructions, procedures, handling, image quality, and diagnostic accuracy may be collected. Based on this feedback, the large language model may be fine-tuned to improve future performance, handling, and diagnostic value. The feedback may be collected in a testing phase or from users during deployment. The feedback may further be collected in free-text format (e.g., a tester or user may freely give feedback) or in a structured manner (e.g., a ranking system per instruction feature). Based on the given feedback, new supervised training cases may be constructed to further improve the performance of method 100. Based on the feedback, also additional algorithms may be put in place to restrict or influence the behavior of the trained large language model. Thereby avoiding a further learning phase for the large language model. For example, a general adversarial network approach may be implemented as a second, independent large language model (e.g., a neural network, a large language model, etc.) to supervise the main model. This supervision large language model could be trained on stricter medical safety information, regulation and guidelines to check the decisions of the main large language model. For example, the supervision large language model may in this manner approve or reject decisions of the main large language model. Thereby providing an extra safety net against undesired and/or unwanted outputs.

A typical reinforcement learning workflow comprises a first step of initializing the large language model with adjustable parameters.

In a second step a reinforcement learning algorithm is applied, wherein the large language model interacts with an environment (e.g., the deployment or testing of the large language model), performs actions based on its current state and parameters (i.e., gives outputs, e.g., instructions, given inputs), and receives rewards or penalties based on the performed actions. For example, the large language model may be used to perform medical imaging examinations (e.g., including instructing, configuring, and operating the medical image acquisition device), and the rewards or penalties based on the performed actions may be based on a user defined performance or based on a pre-defined loss function.

In a third step, the model parameters are adjusted iteratively based on the received rewards or penalties until a predetermined stopping criterion is met.

Upon meeting the stopping criterion, the final large language model algorithm may be configured to make decisions in new states based on the adjusted parameters and learned rewards.

Suitable reinforcement learning algorithms may be any one or a combination of: Q-learning, Deep Q Network, Policy Gradient, or Actor-Critic methods or proximal policy optimization.

For all types of learning, unsupervised, supervised and reinforcement, the adjustable parameters may be determined based on minimizing or maximizing an objective function derived from the received data set.

According to an embodiment, there is further provided a computer program product comprising instructions, which when executed by a processor, cause the processor to perform all of the steps of any of the methods disclosed herein. In one example, the computer program product may be downloadable from a server, e.g., via the internet.

In another example, the computer program product may comprise computer readable instructions or code stored on a (non-transitory) memory or medium, wherein, when a processor executes these instructions, the processor performs any of the herein disclosed methods.

The instructions may be in the form of an executable computer program or script. For example, routines, subroutines and/or functions may be defined in a programming language including but not limited to C, C++, C#, Pascal, BASIC, API calls, HTML, XHTML, XML, ASP scripts, JavaScript, FORTRAN, COBOL, Perl, Java, ADA, .NET, and the like. Instructions may also include instructions for large language, deep learning and/or training of a large language module.

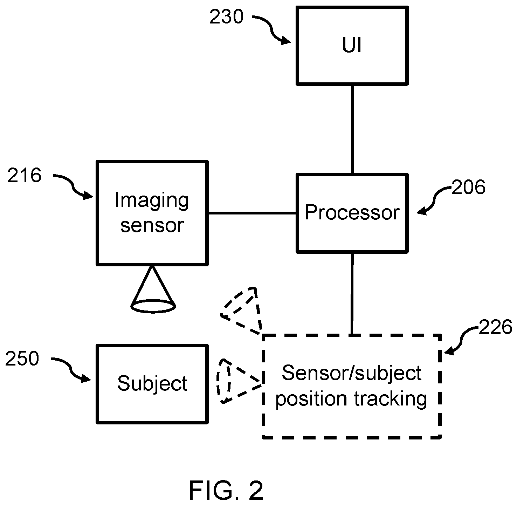

According to an embodiment, there is further provided a medical image acquisition system as shown schematically in Fig. 2, the system comprising:
- a medical image acquisition sensor 216, e.g., an X-ray source and detector, an ultrasound probe comprising a transducer array, etc.;
- a user interface 230, e.g., a (touch)display, a keyboard, a mouse, a microphone, a speaker, etc.; and
- a processor 206 in communication with the medical image acquisition sensor, the subject position tracking device, the medical image acquisition system position tracking device and the user interface, the processor being configured to perform all of the steps of any of the methods disclosed herein.

Optionally, the system may comprise:
- a subject position tracking device 226, e.g., a camera and microphone; and/or
- a medical image acquisition sensor position tracking device 226, e.g., a camera (may be the same as the subject position tracking device or a different camera or other tracking device), inertial measurement units, accelerometers, etc. In some examples, the image acquisition sensor position may be determined with respect to the subject based on acquired medical images.

It should be clear that the medical image acquisition system may be any medical image acquisition system, including but not limited to ultrasound systems, X-ray systems, CT systems and MR systems.

In some embodiments, the medical image acquisition system may further comprise temperature sensors, moisture sensors, pressure sensors, Radar or Lidar technology, and/or any other sensor required to fully track the medical image acquisition system and/or the operator of the medical image acquisition system.

When the medical image acquisition system is an ultrasound system, the ultrasound system may comprise:
- an ultrasound transducer array (e.g., the medical image acquisition sensor) configured to emit and receive acoustic signals that may be used by for example a beam former and a processor to generate ultrasound images.
- a user interface for example comprising a display, speakers, microphones, a keyboard, a mouse, etc. The user interface may for example cart based or a tablet or smartphone in case of a handheld ultrasound probe.
- a pressure sensor configured to determine the pressure between the ultrasound transducer array and a surface of the subject.
- a moisture sensor configured to determine the moisture between the ultrasound transducer array and the surface of the subject.

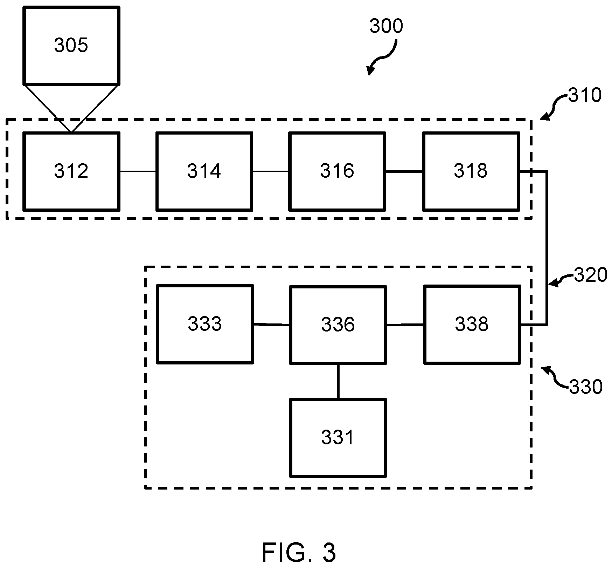

According to an embodiment, the medical image acquisition system may be a (self-operable, smart) ultrasound system 300 as shown in Fig. 3. The smart ultrasound system 300 may comprise an ultrasound probe 310 in communication with a host 330 over a communication interface or link 320. The probe 310 may include a transducer array 312, a beamformer 314, a processor 316, and a communication interface 318. The host 330 may include a user interface 331, a processor 336, a communication interface 338, and a memory 333. The host 330 and/or the processor 336 of the host 330 may also be in communication with other, external types of systems or devices in replacement or addition to the here mentioned systems such as for example, an external memory (e.g., a Picture Archiving and Communication System), external display, a subject tracking system, a probe tracking system, a moisture sensor, a pressure sensor, a temperature sensor, an inertial measurement unit, etc. It is understood that the beamformer may also be a micro-beamformer. It is further understood that the components as shown here may also be configured in alternate arrangements. For example, the processor 316 and/or the beamformer 314 may be located outside of the probe 310 and/or the user interface 331 and/or memory 333 may be located outside of the host 330.

In some examples, the probe 310 is an ultrasound imaging device including a housing configured for handheld operation by a user. The transducer array 312 can be configured to obtain ultrasound data while the user grasps the housing of the probe 310 such that the transducer array 312 is positioned adjacent to or in contact with a subject's skin. The probe 310 is configured to obtain ultrasound data of anatomy within the subject's body while the probe 310 is positioned outside of the subject's body. In some embodiments, the probe 310 can be a patch-based external ultrasound probe. For example, the probe may be a hemodynamic patch. The probe 310 may further comprise a moisture sensor configured to determine the moisture between the transducer array and the subject's skin (i.e., to determine whether enough gel fluid is present). The moisture sensor may also be implemented as a software only solution, wherein the moisture is determined based on an acquired medical image. The probe 310 may further comprise a pressure sensor configured to determine the pressure between the transducer array and the subject's skin. The pressure sensor may also be implemented as a software only solution wherein the pressure exerted by the transducer array on the subject's skin is determined based on an acquired medical image. For example, by determining the deformation of a gel-pad.

The transducer array 312 emits ultrasound signals towards an anatomical object 305 of a subject and receives echo signals reflected from the object 305 back to the transducer array 312. The transducer array 312 can include any suitable number of acoustic elements, including one or more acoustic elements and/or a plurality of acoustic elements. In some instances, the transducer array 312 includes a single acoustic element. In some instances, the transducer array 312 may include an array of acoustic elements with any number of acoustic elements in any suitable configuration. For example, the transducer array 312 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 312 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 312 can be configured to obtain one-dimensional, two-dimensional, and/or three-dimensional images of a subject's anatomy. In some embodiments, the transducer array 312 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The beamformer 314 is coupled to the transducer array 312. The beamformer 314 controls the transducer array 312, for example, for transmission of the ultrasound signals and reception of the ultrasound echo signals. In some embodiments, the beamformer 314 may apply a time-delay to signals sent to individual acoustic transducers within an array in the transducer array 312 such that an acoustic signal is steered in any suitable direction propagating away from the probe 310. The beamformer 314 may further provide image signals to the processor 316 based on the response of the received ultrasound echo signals. The beamformer 314 may include multiple stages of beamforming. The beamforming can reduce the number of signal lines for coupling to the processor 316. In some embodiments, the transducer array 312 in combination with the beamformer 314 may be referred to as an ultrasound imaging component. The beamformer 314 may also be a micro-beamformer.

The processor 316 is coupled to the beamformer 314. The processor 316 may also be described as a processor circuit, which can include other components in communication with the processor 316, such as a memory, beamformer 314, communication interface 318, and/or other suitable components. The processor 316 is configured to process the beamformed image signals. For example, the processor 316 may perform filtering and/or quadrature demodulation to condition the image signals. The processor 316 and/or 336 can be configured to control the array 312 to obtain ultrasound data associated with the object 305.

The communication interface 318 is coupled to the processor 316. The communication interface 318 may include one or more transmitters, one or more receivers, one or more transceivers, and/or circuitry for transmitting and/or receiving communication signals. The communication interface 318 can include hardware components and/or software components implementing a particular communication protocol suitable for transporting signals over the communication link 320 to the host 330. The communication interface 318 can be referred to as a communication device or a communication interface module.

The communication link 320 may be any suitable communication link. For example, the communication link 320 may be a wired link, such as a universal serial bus (USB) link or an Ethernet link. Alternatively, the communication link 320 may be a wireless link, such as an ultra-wideband (UWB) link, an Institute of Electrical and Electronics Engineers (IEEE) 802.11 WiFi link, or a Bluetooth link.

At the host 330, the communication interface 138 may receive signals (e.g., image signals) from the probe 310. The communication interface 338 may be substantially similar to the communication interface 318. The host 330 may be any suitable computing and display device, such as a workstation, a personal computer (PC), a laptop, a tablet, or a mobile phone.

The processor 336 is coupled to the communication interface 338. The processor 136 may also be described as a processor circuit, which can include other components in communication with the processor 336, such as the memory 333, the communication interface 338, and/or other suitable components. The processor 336 can be configured to generate image data from the image signals received from the probe 310. The processor 136 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some examples, the processor 336 can form a three-dimensional (3D) volume image from the image data. In some examples, the processor 336 can perform real-time processing on the image data to provide a streaming video of ultrasound images of the object 305. In some examples, the processor 336 or processor 316 may determine a moisture and/or pressure between the ultrasound probe and the subject's skin based on the imaging data. In other examples, the moisture and/or pressure may be determined from specially designed sensors configured to determine the moisture and pressure between the ultrasound probe 310 and the subject's skin.

The memory 333 is coupled to the processor 336. The memory 333 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 333 may be located within the host 330. There may also be an additional external memory, or an external memory in replacement of memory 333. An external memory may be a cloud-based server or an external storage device, located outside of the host 330 and in communication with the host 330 and/or processor 336 of the host via a suitable communication link as disclosed with reference to communication link 320. Subject information may include measurements, data, files, other forms of medical history, such as but not limited to ultrasound images, ultrasound videos, and/or any imaging information relating to the subject's anatomy. The subject information may include parameters related to an imaging procedure such a probe position and/or orientation.

The user interface 331 is coupled to the processor 336. The user interface 331 is primarily configured to enable interaction with the user (e.g., the operator and/or the subject). The user interface may comprise a (touch screen) display, a speaker, a microphone, a keyboard, a mouse, a camera, etc. The display may be configured to display visual instructions according to method 100, ultrasound images, image videos, and/or any imaging information of the object 305 or instruction material. The speaker may be configured to convey audible information (e.g., instructions) to the operator. The microphone may be configured to receive audible information from the operator (e.g., spoke input). The keyboard may be configured to receive user input in written form.

Method 100 may be implemented in system 300, for example via processor 316 or processor 336, thereby enabling a (smart) ultrasound system. The smart ultrasound system may thus be configured to carry out method 100.

In an example, a user may input user (e.g., the subject in self-operating mode) inputs via the user interface 331 (e.g., in spoken form or written form) comprising any one or a combination of:
- a medical imaging exam type;
- a plurality of symptoms; and
- an instruction to manipulate a (smart) ultrasound system.

In an example, the system 300 or any processor 316, 336 of system 300 may further receive a first plurality of configuration settings of the (smart) ultrasound system, wherein the configuration settings comprise for example imaging settings of an ultrasound probe (e.g., imaging depth, beam steering angle, etc.).

In an example, system 300 or any processor of system 300 may further receive positioning data of the medical imaging sensor with respect to a subject. For example, the system 300 may receive information that the ultrasound probe is placed in an ultrasound probe holder. For example, the system 300 may receive information that the probe is being held by a user based on touch sensors on the ultrasound probe. For example, the system 300 may receive information that the probe is positioned on the subject's skin based on imaging data. For example, the system 300 may receive information that the probe is located at a particular anatomical feature (e.g., heart) based on imaging data. For example, the system 300 may receive information with regard to the probe position with respect to the subject based on a camera.

In an example, system 300 may further receive information with regard to the moisture between ultrasound probe 310 and the subject's skin. Thereby determining whether sufficient gel-fluid is present to acquire ultrasound images of sufficient quality. The moisture information may be received from a moisture sensor or determined based on image quality.

In an example, system 300 may further receive information with regard to the pressure exerted by probe 310 on the subject's skin. The pressure may be determined by a pressure sensor, based on tissue deformation in the imaging data or based on a gel-pad deformation in case a gel-pad is attached to probe 310.

Based on the received inputs of step 101 of method 100, system 300 may further determine outputs 102 according to method 100. In particular, system 300 may determine instructions to operate system 300 to acquire imaging data. For example, system 300 or any processor of system 300 may determine a plurality of instructions to manipulate the medical image acquisition system, wherein the instructions comprise any one or a combination of:
- a second plurality of configuration settings for the (smart) ultrasound system;
   and
- a position guidance for the ultrasound probe with respect to the subject.

In an example, the instructions determined in step 102 are determined based on inputting the received data in a large language model trained according to the herein described training procedures. For example, training data is prepared, the training data comprising:
- a list of symptoms;
- tracking data of an operator performing an ultrasound scan; and
- system data describing the operation of system 300.

The list of symptoms would then comprise the input in a training procedure, while the tracking data of the operator and the system data of system 300 would comprise the associated output. In other words, a large language model is trained to learn the relationship from the ultrasound system settings (e.g., image settings) and the operator actions (e.g., the probe placement) based on for example a given list of symptoms. A large language model trained according to this idea may thus output a series of instructions to operate the system, including placement of the probe, to perform an ultrasound scan. At least some of the instructions may be implemented automatically by the system 300, while at least some instructions may be conveyed to the subject to be implemented (e.g., placing and moving the ultrasound probe). It is however also envisaged by the present invention that the probe may be moved by a robotic arm.

In some examples, the associated output data may further comprise additionally prepared instruction material for each performed ultrasound scan, wherein the instruction material may comprise visual and audible instructions to aid a future operator may be prepared.

In some examples, the associated output data may further comprise an examination report written and/or compiled by the operator.

Using a large language model trained as described herein, system 300 may thus be enabled to be used in a self-operating mode, wherein the subject may perform the ultrasound scan on themselves according to the instructions received from method 100.

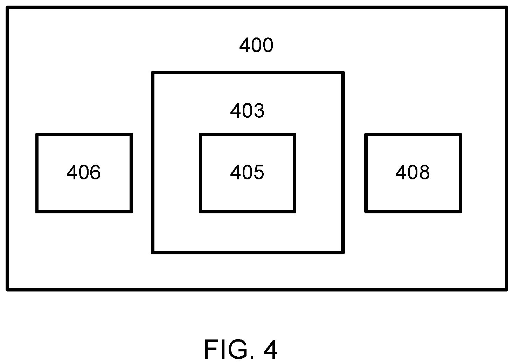

Method 100 may be implemented in any kind of processor circuit. Fig. 4 shows a schematic of a processor circuit 400, according to an embodiment. As shown, the processor circuit 400 may include a processor 406, a memory 403, and a communication module 408. These elements may be in direct or indirect communication with each other, for example via one or more buses.

The processor 406 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 406 may also be implemented as a combination of computing devices, e.g., a combination of a DSP and a microprocessor, a plurality of microprocessors, one or more microprocessors in conjunction with a DSP core, or any other such configuration. The processor 406 may also implement various deep learning networks, which may include a hardware or a software implementation. The processor 406 may additionally include a preprocessor in either hardware or software implementation.

The memory 403 as envisaged by the present disclosure may be any suitable storage device, such as a cache memory (e.g., a cache memory of the processor 406), random access memory (RAM), magneto-resistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processor circuit. In an embodiment, the memory 403 may store instructions 405. The instructions 405 may include instructions that, when executed by a processor 406, cause the processor 406 to perform the operations described herein.

Instructions 405 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 405 may be in the form of an executable computer program or script. For example, routines, subroutines and/or functions may be defined in a programming language including but not limited to Python, C, C++, C#, Pascal, BASIC, API calls, HTML, XHTML, XML, ASP scripts, JavaScript, FORTRAN, COBOL, Perl, Java, ADA, .NET, and the like.

The communication module 408 can include any electronic circuitry and/or logic circuitry to facilitate direct or indirect communication of data between the processor circuit 400, and for example an external display (not shown) and/or an imaging device or system such as an X-ray imaging system or an ultrasound imaging system, etc. In that regard, the communication module 408 can be an input/output (I/O) device. The communication may be performed via any suitable communication technology. For example, the communication may use a wired link, such as a universal serial bus (USB) link or an Ethernet link. Alternatively, the communication may use a wireless link, such as an ultra-wideband (UWB) link, an Institute of Electrical and Electronics Engineers (IEEE) 802.11 WiFi link, or a Bluetooth link.

A clinical workflow example incorporating method 100 is described next. In this clinical workflow, a subject with knee complaints under treatment of an orthopedic physician is considered. A first step when considering knee complaints is typically to take an X-ray image.

According to method 100, an exemplary workflow for an X-ray exam could look as follows:
A subject inputs into an X-ray image acquisition system operating method 100 the symptoms of knee pain and possibly some information about the events of the injury. Additionally or alternatively, the subject may express a desire to have an X-ray image of a left/right knee taken.

Method 100 is thus initiated. Method 100 further receives from the X-ray image acquisition system the remaining inputs received in step 101.

Method 100, based on the received inputs, generates an instruction to the subject to lie on the examination table in a particular manner. Potentially giving visual instructions of a person lying on the examination table, wherein the visual instructions may include a person with a knee facing upward so as to take a frontal view of the knee. The subject positioning and posture may be verified with a camera and further guidance may be given if needed.

Method 100 may then send instructions to an automatic gantry controlling the X-ray image source and/or detector to be positioned to take an X-ray image of the knee. Additionally, or alternatively, method 100 may also give instructions with respect to protective gear to protect for example the crotch part from radiation.

Once the X-ray image source and detector and the subject are positioned, method 100 may control 103 the X-ray system to take an X-ray image. Method 100 may further implement routines to check the image quality and advise on a re-take or to continue to alternative views.

In a next step, method 100 provides new instructions for the subject to alter the positioning and/or posture to for example take a side view of the knee. Method 100 may again verify the subject positioning and/or posture with a camera. Method 100 may also if needed update the X-ray image sensor settings and position before taking the side view of the knee.

Once the front and side view are taken, method 100 may provide an examination report (e.g., an initial findings report) to the subject. This examination report may be used by the subject to determine a potential next step. This findings report may also be sent to the physician treating the subject.

Considering the findings report did not provide any conclusive findings, the physician or the method 100 may recommend a different examination of the knee.

Another exemplary workflow of an ultrasound system according to method 100 could look as follows:
A subject inputs into an ultrasound image acquisition system operating method 100 the symptoms of knee pain with further information that an X-ray image did not provide cues towards the potential cause. Additionally or alternatively, the subject may express a desire to have an ultrasound image of a left/right knee taken.

Method 100 is thus initiated. Method 100 may receive the remaining inputs received in step 101 from the ultrasound system.

Method 100 may, based on the received inputs generate an instruction and conveys this instruction to the subject to sit down on a chair with the knee bend at approximately 90 degrees.

Method 100 may further include instructions, for example audio or visual instructions, to the subject to take an ultrasound probe from an ultrasound probe holder.

Method 100 may further instruct the subject to attach a gel-pad to the ultrasound probe. Method 100 may at every step monitor the subject and the ultrasound probe to verify whether instructions are completed and/or whether the subject requires further assistance to complete the instructions. For example, via a camera.

Method 100 may then instruct the subject to place the probe at a specific location on the knee and to move the probe in a particular manner, for example, a grid pattern to image the entire knee region. Method 100 may verify that the probe is correctly placed via a camera and/or by analyzing the ultrasound images in real-time. Method 100 may verify that the pressure on the knee is sufficient via a pressure sensor. The pressure sensor may be implemented purely in software by determining the gel pad deformation. In case no gel-pad is used, method 100 may verify sufficient gel fluid is used via a moisture sensor. If method 100 detects that for example the pressure exerted was not sufficient, or the image quality is inferior, method 100 may provide further assistance to the subject and repeat imaging.

At the same time as the subject is instructed to place and/or move the probe, method 100 may control 103 the image settings of the ultrasound probe to acquire ultrasound images. Alternatively, method 100 provides instructions to the subject to change the imaging settings.

Method 100 may analyze acquired ultrasound images, and based on the analyzed images determine whether more images are necessary. In some instances, method 100 may also ask for assistance of an experienced operator if after repeated attempts the subject is still not able to take a sufficient quality image.

Once all images are taken, method 100 may generate a medical examination report. The medical examination report may then be provided to the subject and/or the treating physician. For example, the medical examination report may indicate the finding of an inflamed bursa hinting towards patella bursitis.

In these described workflows, it becomes clear that method 100 can, for some imaging procedures, enable full self-operation of the medical image acquisition system by the subject.

It should be noted that the above-mentioned embodiments illustrate rather than limit the invention, and that those skilled in the art will be able to design many alternative embodiments without departing from the scope of the appended claims. It should also be clear that the subject, the operator, the tester and the user may be interchanged. For example, the user in method 100 may also be the subject. The tester in method 100 may also be the user and/or the subject. For example, the operator of a medical image acquisition system may also be a tester, a user and/or a subject. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A method (100) to manipulate a medical image acquisition system comprising an image acquisition sensor, the method comprising using a large language model configured to:
- receive (101) a user input, wherein the user input comprises any one of:
- a medical imaging exam type;
- a plurality of symptoms; and
- an instruction to manipulate the medical image acquisition system; and
- based on the user input, determine (102):
- a first position instruction for the image acquisition sensor with respect to a subject; and
- a first plurality of medical image acquisition system settings, wherein the first plurality of medical image acquisition system settings comprises at least image acquisition sensor settings.

2. The method of claim 1, further comprising:
- conveying the first position instruction for the image acquisition sensor with respect to the subject to a user; and/or
- moving the imaging sensor to the first position.

3. The method of any of the preceding claims, wherein the large language model is further configured to:
- responsive to the image acquisition sensor being positioned at the first position:
- determine an instruction to acquire a medical image;
wherein the method optionally further comprises acquiring (103) the medical image according to the instruction to acquire the medical image.

4. The method of claim 3, wherein the large language model is further configured to:
- responsive to receiving the acquired medical image:
- determine a second plurality of medical image acquisition system settings, wherein the second plurality of medical image acquisition system settings comprises at least image acquisition sensor; and/or
- determine a second position instruction for the image acquisition sensor with respect to the subject.

5. The method of any one of claims 3 to 4, wherein the large language model is further configured to:
- generate (104) a medical examination report.

6. The method of claim 5, wherein the examination report is based on at least any one or a combination of:
- the user input;
- the medical image;
- a medical report template; and
- a medical proficiency level of a potential recipient.

7. The method of any of the preceding claims, wherein the large language model is further configured to:
- responsive to the image acquisition sensor being positioned at the first position:
- receive a pressure signal representing a pressure between the image acquisition sensor and the surface of the subject, and based on the received pressure signal, determine a pressure instruction; and/or
- receive a moisture signal representing moisture at the image acquisition sensor, and based on the received moisture signal, determining a gel fluid application instruction.

8. The method of any of the preceding claims, wherein the large language model is further configured to:
- receive positioning data of the medical image acquisition sensor with respect to the subject; and wherein any one or a combination of the instructions are further based on the positioning data of the medical image acquisition sensor with respect to the subject.

9. The method of any of the preceding claims, wherein the large language model is trained through unsupervised learning, and wherein unsupervised learning comprises:
- receiving an unlabeled data set comprising a plurality of instances, each instance including one or more features; and
- initializing the large language model with adjustable parameters; and
- applying an unsupervised learning algorithm to the data set using the large language model, wherein the unsupervised learning algorithm modifies the adjustable parameters of the large language model based on relationships between the features in the instances without referring to any predetermined label or outcome and wherein the modification of the adjustable parameters is performed iteratively until a stopping criterion is met.

10. The method of any of the preceding claims, wherein the large language model is trained through supervised learning, and wherein supervised learning comprises:
- receiving a labeled dataset comprising a plurality of instances, each instance comprising an input feature and an associated output feature;
- initializing the large language model with adjustable parameters; and
- applying a supervised learning algorithm to the labeled dataset using the large language model, wherein the supervised learning algorithm iteratively modifies the adjustable parameters of the large language model based on a comparison between the large language model prediction given the input feature and the associated output feature until a predetermined stopping criterion is satisfied.

11. The method of claim 10, wherein the labeled data set comprises medical imaging acquisition exams, wherein, in each instance, the input feature comprises:
- the user input; and
- optionally:
- the pressure,
- the moisture and
- the medical image; and
the associated output comprises:
- the first position instruction for the image acquisition sensor with respect to a subject
- the first plurality of medical image acquisition system settings; and
- optionally:
- the instruction to acquire a medical image;
- the second plurality of medical image acquisition system settings;
- the second position instruction for the image acquisition sensor with respect to the subject;
- the medical examination report;
- the pressure instruction;
- the gel-fluid application instruction.

12. The method of any of the preceding claims, wherein the large language model is trained through reinforcement learning, and wherein reinforcement learning comprises:
- initializing the large language model with adjustable parameters;
- applying a reinforcement learning algorithm, wherein the large language model interacts with an environment, performs actions based on its current state and parameters, and receives rewards or penalties based on the performed actions; and
- iteratively adjusting the model parameters based on the received rewards or penalties until a predetermined stopping criterion is met.

13. The method of claim 12, wherein the interaction of the large language model with the environment comprises instructing, configuring, and performing medical imaging examinations according to the method of any of claims 1 to 8, and wherein the rewards or penalties based on the performed actions are based on a user defined performance or based on a pre-defined loss function.

14. A computer program product comprising instructions, which when executed by a processor, cause the processor to perform the method of any of claims 1 to 13.

15. A medical image acquisition system comprising:
- a medical image acquisition sensor (216);
- a user interface (230); and
- a processor (206) in communication with the medical image acquisition sensor and the user interface, the processor being configured to perform the method of any of claims 1 to 13.
